# DEMANDE DE BREVET EUROPEEN

(11) **EP 1 932 493 A1**
(43) Date de publication de la demande: **18.06.2008**
(21) Numéro de dépôt: 07291410.4
(22) Date de dépôt: 27.11.2007
(51) Int. Cl.: A61F 2/24

(54) **Endovalve**

(30) Priorité: 14.12.2006 FR 0610909
(71) Demandeur: Laboratoires Perouse, 60173 Ivry Le Temple (FR)
(72) Inventeur: Styrc Mikolaj, 8190 Kopstal (LU)
(74) Mandataire: Domenego, Bertrand

(57) **Abrégé**

Cette endovalve (10) comprend une endoprothèse (12) déployable entre un état contracté et un état dilaté. L'endoprothèse (12) délimite un conduit intérieur (26) de circulation. L'endovalve (10) comprend une valve (14) placée dans le conduit intérieur (26), déplaçable entre une configuration d'obturation du conduit (26) et une configuration de libération du conduit (26).

L'endovalve (10) comprend des moyens (16) de liaison entre la valve (14) et l'endoprothèse (12). Les moyens de liaison (16) comprennent au moins un organe de liaison intermédiaire (50, 52), lié à l'endoprothèse (12), et comprenant au moins une région déplaçable par rapport à l'endoprothèse (12). La valve (14) est fixée sur la région déplaçable.

## Description

La présente invention concerne une endovalve, du type comprenant :
- une endoprothèse déployable entre un état contracté et un état dilaté, l'endoprothèse délimitant un conduit intérieur,
- une valve placée dans le conduit intérieur, déplaçable entre une configuration d'obturation du conduit intérieur et une configuration de libération du conduit intérieur ; et
- des moyens de liaison entre la valve et l'endoprothèse.

De telles endovalves sont destinées à remplacer notamment des valves natives déficientes dans le coeur humain.

On connaît une endovalve du type précité, qui est implantée en remplacement d'une valve native par la voie endoluminale. A cet effet, l'endovalve est amenée jusqu'à son point d'implantation dans un état contracté, par exemple dans un cathéter. Puis, l'endovalve est déployée en regard de la valve native devant être remplacée où elle se plaque contre la paroi délimitant la valve native.

Pour assurer un bon fonctionnement de la valve portée par l'endovalve, il est nécessaire que celle-ci soit solidement fixée sur l'endoprothèse qui constitue la structure d'appui contre la paroi.

Par suite, il est connu de coudre la valve dans l'endoprothèse en la fixant solidement sur les fils constituant le treillis déployable de l'endoprothèse. Cette couture est réalisée hors du corps humain, lorsque l'endoprothèse occupe son état dilaté de manière homogène.

Toutefois, la forme de l'endoprothèse après son implantation dépend de la morphologie du patient dans lequel elle est implantée. Ainsi, si la configuration de la valve native ne présente pas une section transversale cylindrique, l'endoprothèse occupe alors dans son état dilaté une section qui peut être elliptique, voire même triangulaire. De telles formes non homogènes radialement créent des contraintes sur la valve fixée sur le treillis, ce qui tend à détériorer la valve ou à altérer son fonctionnement.

Un but de l'invention est donc d'obtenir une endovalve pouvant être implantée de manière satisfaisante dans des patients présentant des morphologies différentes, sans nuire à la fiabilité de son fonctionnement.

A cet effet, l'invention a pour objet une endovalve du type précité caractérisée en ce que les moyens de liaison comprennent au moins un organe de liaison intermédiaire, lié à l'endoprothèse et comprenant au moins une région déplaçable par rapport à l'endoprothèse, la valve étant fixée sur la ou sur au moins une région déplaçable.

L'endovalve selon l'invention peut comprendre l'une ou plusieurs des caractéristiques suivantes, prise(s) isolément ou suivant toutes combinaisons techniquement possibles :
- l'endoprothèse comprend une paroi latérale délimitant au moins une ouverture transversale débouchant dans le conduit intérieur et à l'extérieur de l'endoprothèse à travers la paroi latérale, l'assemblage formé par l'organe de liaison intermédiaire et la valve étant engagé à travers l'ouverture transversale ;
- l'assemblage formé par la valve et l'organe de liaison intermédiaire est mobile localement par rapport à la paroi latérale suivant une course délimitée par l'ouverture transversale ;
- l'organe de liaison intermédiaire comprend une région extérieure placée à l'extérieur de l'endoprothèse et une région intermédiaire raccordant la ou chaque région déplaçable à travers une ouverture transversale de l'endoprothèse ;
- l'organe de liaison intermédiaire est mobile localement suivant toute sa longueur par rapport à l'endoprothèse ;
- l'organe de liaison intermédiaire est placé à l'extérieur de l'endoprothèse, la valve étant fixée sur l'organe de liaison intermédiaire à travers au moins une ouverture transversale ménagée dans l'endoprothèse le long d'une ligne de fixation ;
- l'organe de liaison intermédiaire présente une forme sensiblement complémentaire à la forme de la ligne de fixation ;
- l'organe de liaison intermédiaire est un lien filiforme ;
- l'organe de liaison intermédiaire est un ruban ; et
- l'organe de liaison intermédiaire est un manchon tubulaire placé autour de l'endoprothèse.

L'invention sera mieux comprise à la lecture de la description qui va suivre, donnée uniquement à titre d'exemple et faite en se référant aux dessins annexés, sur lesquels :
- la figure 1 est une vue de face d'une première endovalve selon l'invention ;
- la figure 2 est une vue prise en coupe suivant un plan transversal horizontal II de l'endovalve de la figure 1 ;
- la figure 3 est une vue analogue à la figure 1 d'une deuxième endovalve selon l'invention ;

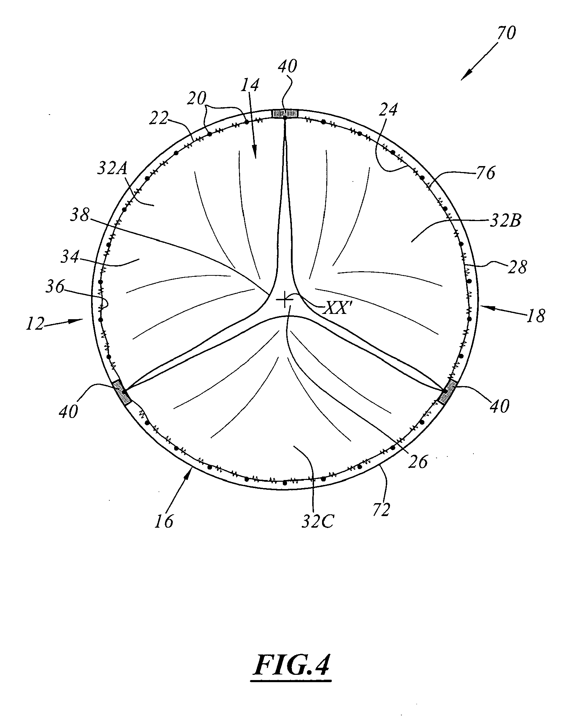

- la figure 4 est une vue analogue à la figure 2 de la deuxième endovalve selon l'invention ;
- la figure 5 représente deux exemples de rubans intermédiaires de fixation pour la deuxième valve selon l'invention ;
- la figure 6 est une vue analogue à la figure 1 d'une troisième endovalve selon l'invention ; et
- la figure 7 est une vue analogue à la figure 2 de la troisième endovalve selon l'invention.

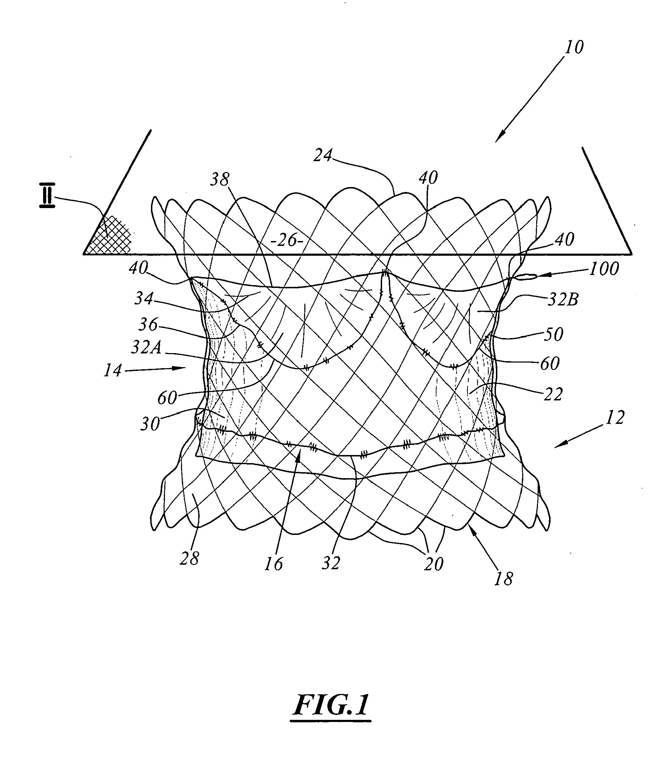

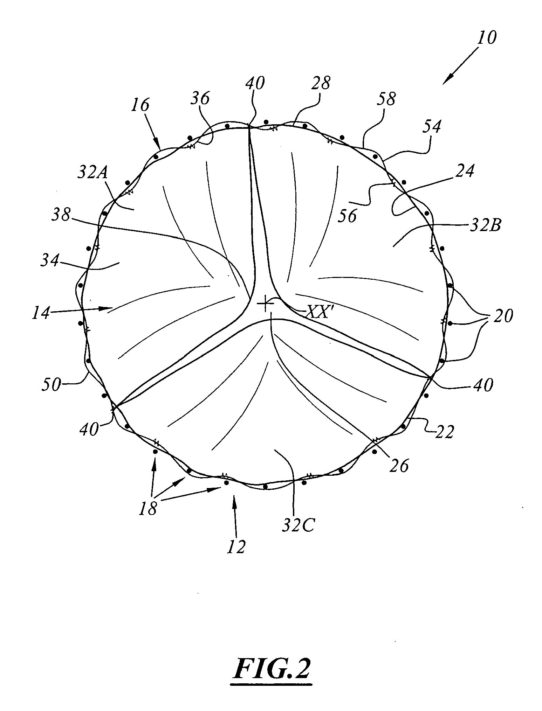

La première endovalve 10 selon l'invention, représentée sur les figures 1 et 2, est destinée au remplacement d'une valve cardiaque native déficiente par la voie endoluminale.

L'endovalve 10 comprend une endoprothèse tubulaire 12, une valve 14 placée dans l'endoprothèse 12, et des moyens 16 de liaison intermédiaire entre l'endoprothèse 12 et la valve 14.

L'endoprothèse tubulaire 12 est formée par une armature constituée d'un treillis tubulaire 18 qui possède des propriétés de ressort. Le treillis 18 est obtenu par tressage d'au moins un fil d'acier inoxydable, d'un alliage à mémoire de formes, ou d'un polymère. En variante, le treillis est obtenu par découpe laser d'un tube.

Le treillis 18 comprend une pluralité de segments filiformes 20 se croisant et délimitant des ouvertures transversales 22 en forme de losange.

Le treillis 18 définit une surface intérieure 24 délimitant un conduit intérieur 26 de circulation du sang, et une surface extérieure 28 destinée à être appliquée contre la paroi d'un vaisseau sanguin, en regard de la valve native déficiente.

Les ouvertures transversales 22 débouchent dans la surface intérieure 24 et dans la surface extérieure 28.

Le treillis métallique 18 de l'endoprothèse 12 est déployable entre un état contracté dans lequel il présente un petit diamètre, et un état dilaté, constituant son état de repos, dans laquelle il présente un grand diamètre.

Dans l'exemple représenté sur la figure 1, le treillis 18 est déployable spontanément entre son état contracté et son état dilaté. En variante, un ballon est utilisé pour le déployer.

La valve 14 est par exemple réalisée à base d'une valve native d'un animal comme un porc. En variante, elle est réalisée à base de tissus naturels ou synthétiques.

Elle comprend une base tubulaire 30 prolongée vers le haut par trois feuillets 32A, 32B, 32C d'obturation du conduit 26.

La base 30 présente une surface extérieure sensiblement complémentaire à la surface intérieure 24 de l'endoprothèse 12, sur laquelle elle est appliquée.

Dans l'exemple représenté sur la figure 2, les feuillets 32A, 32B, 32C sont venus de matière avec la base 30 qu'ils prolongent vers le haut.

Les feuillets 32A, 32B, 32C sont répartis autour de l'axe X-X' du conduit 26. Chaque feuillet 32A, 32B, 32C occupe ainsi un secteur angulaire de 120° environ autour de l'axe X-X'.

Chaque feuillet 32A, 32B, 32C est formé par une membrane en forme de poche 34 s'étendant en regard d'une partie de la surface intérieure 24. Chaque feuillet 32A, 32B, 32C est délimité vers l'extérieur et vers le bas par une ligne de fixation 36 sensiblement en forme de U, et vers le haut par un bord libre 38 mobile dans le conduit 26.

La ligne de fixation 36 s'étend sensiblement sur la surface intérieure 24 de l'endoprothèse 12 jusqu'à deux extrémités supérieures 40 formant les extrémités latérales du bord libre 38. Chaque extrémité supérieure 40 d'un feuillet 32A, 32B, 32C est commune à deux feuillets 32A, 32B, 32C adjacents.

Comme on le verra plus bas, les lignes de fixation 36 sont cousues exclusivement sur les moyens de liaison intermédiaires 16 et non sur le treillis 18 de l'endoprothèse 12.

Le bord libre 38 s'étend sensiblement horizontalement dans le conduit intérieur 26 entre les extrémités supérieures 40.

Le bord libre 38 et les poches 34 sont déplaçables sous l'effet de la pression sanguine dans le conduit intérieur 26 entre une configuration de libération du conduit 26 (non représentée) et une configuration d'obturation du conduit 26 représentée sur la figure 2.

Dans la configuration de libération, les poches 34 sont plaquées contre la surface intérieure 24 de l'endoprothèse 12. Les bords libres 38 s'étendent alors le long de la surface 24 à l'écart les uns des autres.

La distance qui sépare chaque bord libre 38 de l'axe X-X' est alors maximale. La surface occupée par les feuillets 32A, 32B, 32C dans le conduit 26 est alors minimale pour permettre le passage du sang.

Dans la configuration d'obturation, les poches 34 sont déployées à l'intérieur du conduit 26. Les bords libres 38 sont situés au voisinage de l'axe X-X'. Le bord libre 38 de chaque feuillet 32A, 32B, 32C est appliqué sur sensiblement toute sa longueur contre le bord libre 38 des feuillets 32B, 32C, 32A adjacents.

Dans cette configuration, les poches 34 occupent une surface maximale dans le conduit 26 et le passage du sang dans le conduit 26 est sensiblement empêché.

Selon l'invention, les moyens de liaison 16 entre la valve 14 et l'endoprothèse 12 sont formés par des organes de liaison intermédiaires liés à l'endoprothèse 12, mais comprenant au moins une région déplaçable par rapport à cette endoprothèse 12.

Dans la première endovalve 10 selon l'invention, les moyens de liaison 16 sont formés par un fil de liaison inférieur 50, pour la fixation des feuillets 32A, 32B, 32C et par un fil de liaison inférieur 52 pour la fixation de la base 30.

Les fils 50, 52 sont entrelacés dans le treillis 18 de l'endoprothèse 12 entre la surface intérieure 24 et la surface extérieure 28. Les fils 50, 52 passent ainsi successivement à l'intérieur et à l'extérieur des segments filiformes 20 constituant le treillis 18 à travers les ouvertures 22.

Les fils 50, 52 sont donc liés à l'endoprothèse en ce qu'ils sont déplaçables conjointement avec cette endoprothèse 12 lorsque l'endoprothèse 12 est déplacée sur une grande distance.

Toutefois, les fils 50, 52 sont déplaçables localement sur toute leur longueur par rapport à l'endoprothèse 12, sur une course délimitée par l'étendue de chaque ouverture 22 dans laquelle le fil 50, 52 est engagé.

Chaque fil 50, 52 définit une pluralité de régions extérieures 54 situées à l'extérieur de l'endoprothèse 12, une pluralité de régions intérieures 56 de fixation placées dans le conduit intérieur 26, et une pluralité de régions intermédiaires 58, chaque région intermédiaire 58 reliant une région 54 à une région 56 à travers une ouverture 22 transversale.

Le fil de liaison supérieur 50 présente trois pans 60 en forme de U s'étendant respectivement en regard des lignes de fixation 36 de chaque feuillet 32A, 32B, 32C.

Les feuillets 32A, 32B, 32C sont cousus sur les régions intérieures 56 de fixation. Les feuillets 32A, 32B, 32C et le fil 50 forment ainsi un assemblage solidaire engagé dans une pluralité d'ouvertures transversales 22.

De même, le fil de liaison inférieur 50 s'étend le long du bord inférieur de la base 30 suivant une circonférence de la surface intérieure 24 prise autour de l'axe X-X'. La base 30 de la valve 14 est cousue sur les régions intérieures 56 de fixation du fil de liaison inférieur 50, le long de cette circonférence.

La valve 14 est ainsi globalement fixe par rapport à l'endoprothèse tubulaire 12 pour être déplacée conjointement avec cette endoprothèse 12 par coopération entre le treillis 18 et l'assemblage formé par la valve 14 et les fils intermédiaires 50, 52 de liaison. L'assemblage formé par la valve 14 et les fils 50, 52 est en outre déplaçable localement par rapport à l'endoprothèse 12 dans les ouvertures 22 suivant une course délimitée par l'étendue de chaque ouverture 22.

Le fonctionnement de la première endovalve 10 selon l'invention va maintenant être décrit.

Initialement, lors de la fabrication de l'endovalve 10, les fils 50, 52 sont engagés dans le treillis 18 de l'endoprothèse 12 pour former respectivement trois pans 60 en forme de U autour de l'axe X-X' et pour s'étendre le long d'une circonférence inférieure du treillis 18. Puis, la valve 14 est introduite dans le conduit 26 et est cousue sur les régions intérieures 56 de fixation des fils 50, 52.

L'endoprothèse 12 est ensuite rétractée dans son état contracté et maintenue dans cet état par des moyens de largage (non représentés) de l'endovalve 10 dans l'organisme, constitués par exemple par un fourreau extérieur ou par un système de largage à fils comme décrit dans la demande FR-A- 2 863 160 de la Demanderesse.

Puis, les moyens de largage transportant l'endovalve 10 sont introduits dans le patient et amenés vers le lieu d'implantation, par exemple par la voie endoluminale.

Lorsque l'endovalve 10 est située en regard de son lieu d'implantation, au voisinage de la valve native, les moyens de largage sont libérés pour faire passer l'endovalve 10 de son état contracté à son état dilaté. La surface extérieure 28 de l'endoprothèse 12 est alors appliquée contre la paroi d'un conduit de circulation du sang. L'endoprothèse 12 présente alors une section transversale sensiblement complémentaire de celle du conduit dans laquelle elle est appliquée.

Toutefois, même si le treillis 18 ne se déploie pas de manière homogène autour de l'axe X-X', c'est-à-dire que certaines ouvertures transversales 22 du treillis 18 sont plus déformées que d'autres, les contraintes éventuelles sur la valve 14 se relâchent par déplacement local des fils 50, 52 et de la valve 14 par rapport au treillis 18 dans les ouvertures 22. La valve 14 occupe ainsi un état relâché, ce qui augmente sa fiabilité en fonctionnement au cours du temps.

De plus, la valve 14 étant fixée exclusivement sur les fils 50,52 de liaison intermédiaires sans être fixée directement sur les segments filiformes 20 constituant le treillis 18, le déploiement de ce treillis 18 lors du passage de l'état contracté à l'état dilaté de l'endoprothèse 12, n'est pas gêné par la présence de la valve 14, ce qui améliore la facilité du largage de l'endoprothèse 12.

Dans une variante, les fils 50, 52 sont placés autour de la surface extérieure 28 de l'endoprothèse 12. Les fils 50, 52 sont fixés ponctuellement au treillis 18, par exemple en au moins trois points autour de la périphérie.

La valve 14 est fixée aux fils 50, 52 à travers les ouvertures transversales 22 dans les régions libres des fils 50, 52 situées entre leurs points de fixation sur le treillis 18.

Dans une autre variante, la valve 14 comprend une paroi tubulaire entourant et portant les feuillets 32A, 32B, 32C. La paroi tubulaire est appliquée contre la surface intérieure 24.

Dans ce cas, le fil supérieur 50 présente un contour sensiblement circulaire, comme le fil intérieur 52. La paroi tubulaire de la valve 14 est alors fixée sur les fils 50, 52.

La deuxième endovalve 70 selon l'invention diffère de la première endovalve 10 en ce que les moyens de liaison 16 comprennent des organes intermédiaires formés par des rubans de tissu 72, 74 de formes sensiblement complémentaires à celles des lignes de couture 36 de la valve 14. Ainsi, le ruban de fixation supérieur 72 présente trois pans 60 en forme de U cousus entre eux par leurs extrémités supérieures. Le ruban inférieur 74 présente une forme d'anneau.

Les rubans 72, 74 sont par exemple réalisés à base d'un tissu biologiquement compatible tissé ou tricoté comme du Dacron.

Les rubans 72, 74 sont appliqués contre la surface extérieure 28 de l'endoprothèse 12, à l'extérieur du conduit intérieur 26 et sont reliés à la valve 14 par des coutures 76 réalisées au travers des ouvertures 22.

Le fonctionnement de cette deuxième endovalve 70 est par ailleurs sensiblement analogue à celui de la première endovalve 10.

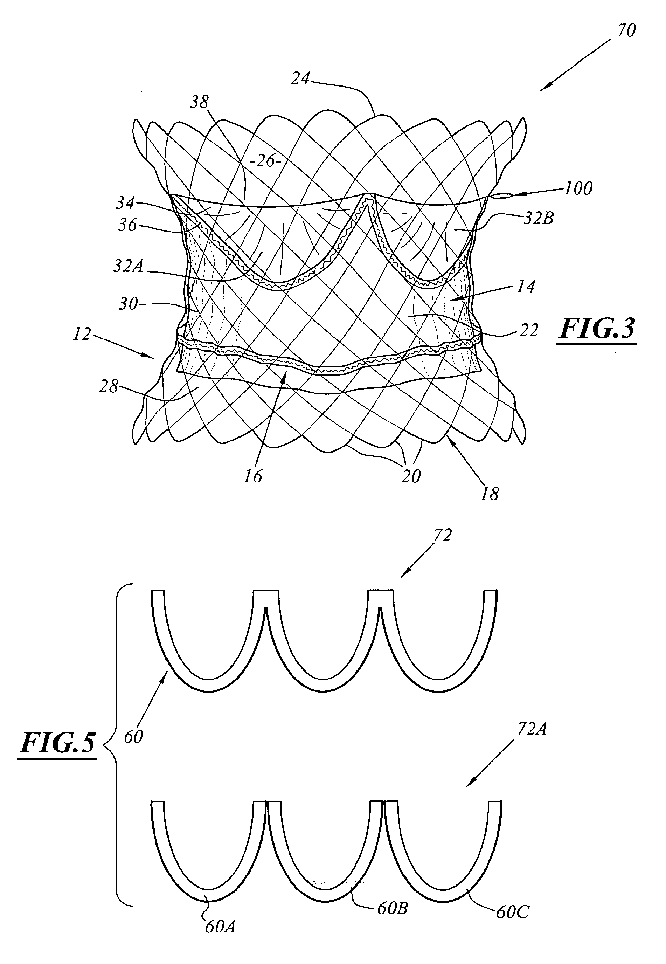

Dans une variante représentée partiellement sur la figure 5, le ruban de fixation 72A comprend trois pans 60A, 60B, 60C indépendants, libres l'un par rapport à l'autre. Chaque pan 60A, 60B, 60C est fixé respectivement à la ligne de fixation 36 d'un feuillet 32A, 32B, 32C respectif.

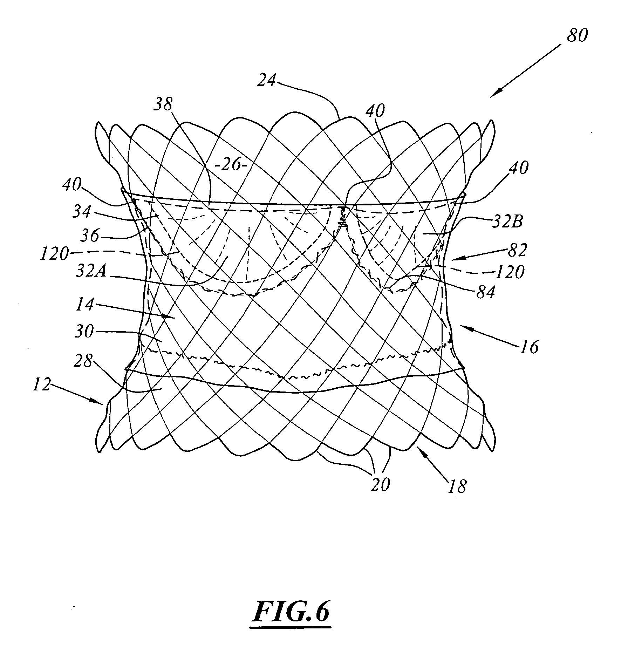

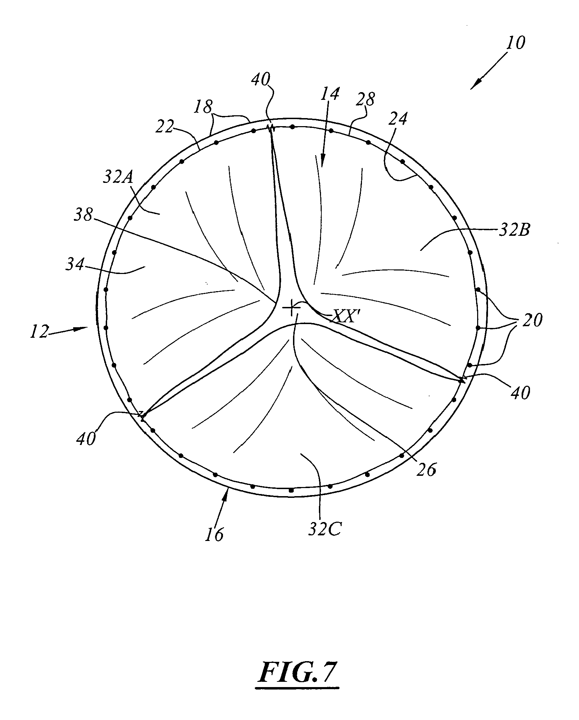

La troisième endovalve 80 selon l'invention, représentée sur les figures 6 et 7 diffère de la deuxième endovalve 70 en ce que l'organe intermédiaire de liaison des moyens de liaison 16 est formé par un manchon unique 82 de tissu engagé autour de la surface extérieure 28 de l'endoprothèse 12.

Le manchon 82 présente une hauteur sensiblement égale à celle de la valve 14. Des coutures 84 fixent solidairement les feuillets 32A, 32B, 32C et la base 30 sur le manchon 84 à travers les ouvertures transversales 22.

Le fonctionnement de cette troisième endovalve 80 selon l'invention est par ailleurs analogue à celui de la première endovalve 10 ou de la deuxième endovalve 70.

Dans une variante représentée en pointillés sur la Figure 6, le manchon 82 définit trois encoches 120 en forme de U s'étendant chacune respectivement en regard d'un feuillet 32A, 32B, 32C, au-dessus des lignes de fixation 36 des feuillets 32A, 32B, 32C. Les encoches 120 débouchent vers le haut dans le bord supérieur du manchon 82, afin de permettre au sang de déplacer les feuillets 32A, 32B, 32C depuis leur configuration de libération plaquée contre la surface intérieure 24, vers leur configuration d'obturation du conduit 26.

Dans une variante représentée en pointillés sur la figure 1, le fil supérieur 52 est muni d'un passant 100 faisant saillie extérieurement par rapport à la surface 28 pour permettre la récupération de l'endovalve 10 par traction du fil 10 comme décrit dans la demande de brevet en France n°06 02932 de la Demanderesse.

## Revendications

1. Endovalve (10 ; 70 ; 80), du type comprenant :
- une endoprothèse (12) déployable entre un état contracté et un état dilaté, l'endoprothèse (12) délimitant un conduit intérieur (26),
- une valve (14) placée dans le conduit intérieur (26), déplaçable entre une configuration d'obturation du conduit intérieur (26) et une configuration de libération du conduit intérieur (26) ; et
- des moyens (16) de liaison entre la valve (14) et l'endoprothèse (12) ;
**caractérisée en ce que** les moyens de liaison (16) comprennent au moins un organe (50, 52 ; 72, 74 ; 82) de liaison intermédiaire, lié à l'endoprothèse (12) et comprenant au moins une région (56) déplaçable par rapport à l'endoprothèse (12), la valve (14) étant fixée sur la ou sur au moins une région déplaçable (56).

2. Endovalve (10 ; 70 ; 80) selon la revendication 1, **caractérisé en ce que** l'endoprothèse (12) comprend une paroi latérale (18) délimitant au moins une ouverture transversale (22) débouchant dans le conduit intérieur (26) et à l'extérieur de l'endoprothèse (12) à travers la paroi latérale (18), l'assemblage formé par l'organe de liaison intermédiaire (50, 52 ; 72, 74 ; 82) et la valve (14) étant engagé à travers l'ouverture transversale (22).

3. Endovalve (10 ; 70 ; 80) selon la revendication 2, **caractérisé en ce que** l'assemblage formé par la valve (14) et l'organe de liaison intermédiaire (50, 52 ; 72, 74 ; 82) est mobile localement par rapport à la paroi latérale (18) suivant une course délimitée par l'ouverture transversale (22).

4. Endovalve (10 ; 70 ; 80) selon l'une quelconque des revendications 2 ou 3, **caractérisé en ce que** l'organe de liaison intermédiaire (50, 52 ; 72, 74 ; 82) comprend une région extérieure (54) placée à l'extérieur de l'endoprothèse (12) et une région intermédiaire (58) raccordant la ou chaque région déplaçable (56) à travers une ouverture transversale (22) de l'endoprothèse.

5. Endovalve (10 ; 70 ; 80) selon l'une quelconque des revendications précédentes, **caractérisé en ce que** l'organe de liaison intermédiaire (50, 52 ; 72, 74 ; 82) est mobile localement suivant toute sa longueur par rapport à l'endoprothèse (12).

6. Endovalve (70 ; 80) selon l'une quelconque des revendications précédentes, **caractérisé en ce que** l'organe de liaison intermédiaire (72, 74 ; 82) est placé à l'extérieur de l'endoprothèse (12), la valve (14) étant fixée sur l'organe de liaison intermédiaire (72, 74 ; 82) à travers au moins une ouverture transversale (22) ménagée dans l'endoprothèse (12) le long d'une ligne de fixation (36).

7. Endovalve (70) selon la revendication 6, **caractérisé en ce que** l'organe de liaison intermédiaire (72, 74) présente une forme sensiblement complémentaire à la forme de la ligne de fixation (36).

8. Endovalve (10) selon l'une quelconque des revendications précédentes, **caractérisé en ce que** l'organe de liaison intermédiaire (50, 52) est un lien filiforme.

9. Endovalve (70) selon l'une quelconque des revendications précédentes, **caractérisé en ce que** l'organe de liaison intermédiaire (72, 74) est un ruban.

10. Endovalve (80) selon l'une quelconque des revendications précédentes, **caractérisé en ce que** l'organe de liaison intermédiaire (82) est un manchon tubulaire placé autour de l'endoprothèse (12).
